# DEMANDE DE BREVET EUROPEEN

(11) **EP 0 943 298 A2**
(43) Date de publication de la demande: **22.09.1999**
(21) Numéro de dépôt: 99400703.7
(22) Date de dépôt: 22.03.1999
(51) Int. Cl.: A61F 2/00, A61L 31/00

(54) **Plaque chirurgicale**

(30) Priorité: 20.03.1998 FR 9803418
(71) Demandeur: Cousin Biotech (S.A.S.), 59117 Wervicq Sud (FR)
(72) Inventeur: Frismand, Jean, 59700 Marcq en Baroeul (FR); Solecki, Gilles, 59100 Roubaix (FR)
(74) Mandataire: Breese, Pierre

(57) **Abrégé**

L'invention concerne une plaque chirurgicale souple composée d'un complexe multicouche comprenant une surface favorisant l'adhérence d'un tissu biologique et une surface non adhérente. Elle est constituée par une couche extérieure constituée par un textile tissé macroporeux, tricoté ou non-tissé, ou grille extrudé à l'exclusion d'un matériau uniforme perforé, revêtu par une surface non adhérente.

L'invention concerne également 2 procédé de préparation de telle plaque.

## Description

La présente invention a pour objet une plaque chirurgicale souple composite destinée à être implantée dans l'organisme afin de compenser la défaillance de certains tissus et en particulier, mais non exclusivement, en tant que plaque neurochirurgicale ou comme plaque de contention, par exemple en cas d'hernies inguinales et d'éventrations.

Des plaques de ce genre sont connues et décrites, par exemple de FR-A-2 712 176 et FR-A-2 712 177. En raison de leur fonction, elles doivent présenter une face lisse qui sera, après pose, la face interne et une couche externe poreuse qui constituera la face externe, la porosité permettant l'accrochage des tissus organiques pour compenser les effets de glissement relatifs de la plaque et des tissus adjacents.

Or, il est difficile de fixer une couche lisse non adhérente sur une face poreuse.

On connaît dans l'état de la technique le brevet PCT WO93/17635. Ce document divulgue une prothèse composite formée par un matériau tissé et un film de faible adhésion, réunis par des zones de collage formant une matrice ou des serpentins adhésifs. Un tel procédé de fabrication n'est pas totalement satisfaisant, car l'épaisseur des zones de collage et difficile à maîtriser, et nécessite des moyens de dépose de colle précis, pilotés par ordinateur, et donc coûteux. Par ailleurs, les zones de collages ainsi réalisées altèrent les qualités mécaniques de la structure composite ainsi réalisée, qui ne présente pas les qualités de finesse et de souplesse requises pour des applications chirurgicales.

On connaît également le brevet WO97/35533 divulguant une prothèse, notamment d'une plaque de réparation pour hernie formée par l'association d'un film réalisé en un matériau favorisant l'adhérence d'un tissu biologique, et d'une couche d'un matériau empêchant l'adhésion des tissus biologiques. Ce brevet concerne une prothèse à mailles qui comprend une première couche extérieure et une seconde couche extérieure qui est associée à la première couche extérieure en y étant opposée. La première couche extérieure est conçue pour stimuler la fibroblaste. Le matériau de la première couche extérieure est une polyoléfine, telle que le polypropylène. La seconde couche extérieure est constituée d'un matériau qui empêche la fibroplasie et qui est un polymère fluoré, tel que le polytetrafluoroethylene (PTFE). L'éthylène-propylène fluoré (EPF), le tetrafluoroéthylène (TFE) et l'éthylène-tetrafluoroethylene (ETFE) sont d'autres polymères fluorés qui peuvent servir a fabriquer ladite seconde couche extérieure.

Un autre brevet, le brevet WO94/19029 divulgue une prothèse comprenant une feuille macro poreuse en PTFE, présentant des trous visibles à l'oeil nu, et une feuille micro poreuse, associées par laminage. Ce brevet se rapporte à une feuille de matière destinée à servir de pièce implantable pour la réparation de tissus vivants, cette feuille se présentant sous la forme d'une couche de matière en feuille de polytétrafluoroéthylène poreux déposée en strates sur une couche de matière en feuille du type à mailles traversée par une multitude d'ouvertures, lesquelles ont un diamètre minimum moyen d'environ 0,1 mm.

L'objet de l'invention est de proposer une prothèse améliorée comportant un support formé par un textile, revêtu par un matériau présentant une surface non adhérente. On entendra par 〈〈 textile 〉〉 au sens du présent brevet un matériau macroporeux tissé, tricoté, non-tissé, (c'est-à-dire formé par agglomération de fibres sans entrecroisement régulier des fibres ou grilles extrudées). Le terme de 〈〈 textile 〉〉 n'englobera toutefois pas, au sens du présent brevet, un film uniforme micro perforé.

À cet effet, l'invention concerne une plaque chirurgicale composée d'un complexe multicouche comprenant une face interne non adhérente et une couche externe textile, caractérisée en ce que la couche de liaison est constituée par un film continu de silicone.

L'idée de base de la présente invention réside dans la formation d'une surface non adhérente sur un support textile.

Selon une première variante, le textile est constitué par un matériau tissé ou non tissé présentant des trous de l'ordre de 0,1 à 0,3 mm.

Selon un mode de réalisation préféré, le support est constitué par un matériau tricoté présentant une porosité comprise entre 0,5 et 2 mm.

Selon un premier mode de réalisation, la surface non-adhérente est réalisée par un film de silicone déposé par transfert sur la surface du support.

Selon un deuxième mode de réalisation, la surface non-adhérente est constituée par un composé fluoré liée au support textile par une couche de liaison uniforme constituée par un polymère. La prothèse selon ce deuxième mode de réalisation est alors obtenue en réunissant les deux couches par une couche de liaison appropriée, permettant de fixer sur un matériau poreux tissé, tricoté ou non-tissé un film très fin d'un matériau anti-adhérent.

Selon un mode de réalisation particulier, la surface non adhérente est réalisée par collage d'une feuille de polymère fluoré sur une structure textile à l'aide d'un polymère thermoplastique ou thermodurcissable.

De préférence, la couche externe est une couche de textile fibrillaire non-tissée permettant une bonne colonisation par les tissus organiques recouvrant la plaque ou patch.

Selon une variante avantageuse, le support textile est formé par une association d'un matériau résorbable et d'un matériau non résorbable.

La couche de liaison entre les couches internes et externe est constituée par un polymère qui peut polymériser ou réticuler à une température comprise entre 0 et 230°C et pendant une durée inversement proportionnelle à la température. De préférence, le polymère est constitué par un silicone polymérisant à une température modérée.

Le composé fluoré est de préférence du polytétrafluoroéthylène PTFE expansé ou non, ce composé présentant des caractéristiques connues de coefficient d'adhérence relativement faible, ce qui permet un débattement de la plaque par rapport à l'organe recouvert.

L'invention concerne également un procédé de fabrication d'une plaque chirurgicale multicouche, consistant à déposer sur un support textile une couche non adhérente.

D'autres caractéristiques et avantages de la présente invention apparaîtront au cours de la description qui va suivre d'un mode particulier de réalisation, donné uniquement à titre d'exemple non limitatif, en regard des dessins annexés où :
- la figure 1 représente, en perspective, une plaque chirurgicale selon l'invention ;
- les figures 2 et 3 représentent une vue en coupe d'une plaque selon l'invention à deux étapes de fabrication ;
- les figures 4 et 5 représentent une vue en coupe d'un autre type de plaque selon l'invention à deux étapes de fabrication.

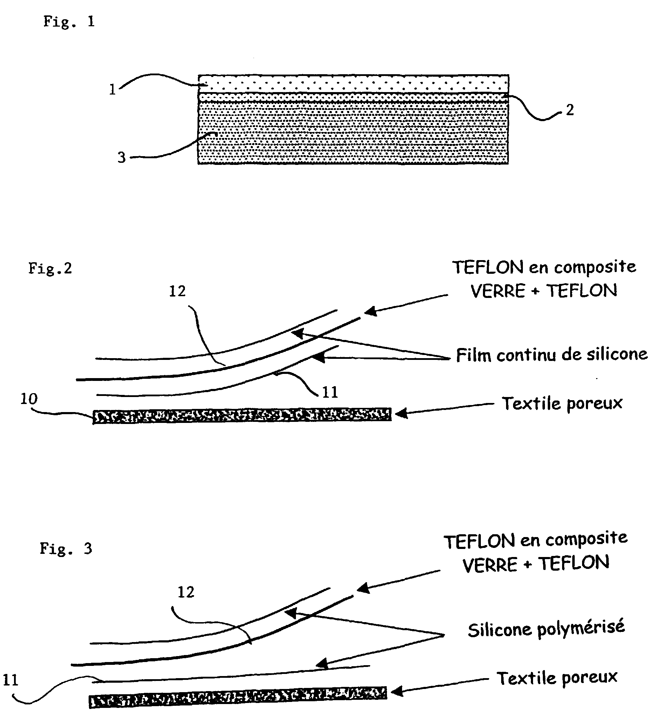

Sur la figure 1, on voit que la plaque se compose d'une couche supérieure (1) de polymère fluoré tel que du 〈〈 Téflon 〉〉 (Marque déposée) ou du 〈〈 Gore-tex 〉〉 (Marque déposée) microporeux qui constituera la couche interne, une couche textile (2) par exemple en polypropylène ou un polyamide biocompatible, et d'une couche de liaison (3) constituée par du silicone.

Les figures 2 et 3 représentent une plaque, réalisée selon une première technique de fabrication consistant à transférer un film de silicone (11) sur un support textile (10).

Le procédé décrit permet de réaliser un film silicone (11) très fin donc très souple, continu et lisse donc anti-adhérent (épaisseur 100 à 400 micromètres) fixé sur un tissu (tissé, tricot, non tissé) macroporeux (10).

Le tissu (10) présente une porosité fonction du matériau employé, par exemple : pour un non tissé, des trous de 0,1 à 0,3mm et pour un tricot des trous de 0,5 à 2mm.

On prépare ensuite une solution de silicone en dispersion dans du trichloroéthane 1,1,1 à raison de 60 grs de silicone pour 100 cm3 de trichloréthane. La silicone employée est de marque NUSIL type MED 42-11 par exemple et présente deux parties miscibles.

L'étape suivante consiste à tremper une feuille composite VERRE + TEFLON (12) de marque commerciale FLUOROCOMPOSITE ou BALTEX d'une épaisseur de 250 micromètres dans la solution réalisée à l'étape précédente afin d'obtenir après évaporation du trichloréthane 1,1,1 un film continu (11) d'environ 300 micromètres de silicone. La feuille fluorocomposite (12) de 250 micromètres présente une facilité de trempage dans un bac de silicone et se décolle ensuite facilement après polymérisation. Mais on pourrait utiliser également une plaque de téflon plus épaisse (voire 1cm).

On dépose ensuite la feuille composite (12) enduite de silicone sur un textile poreux (tissu, tricot ou non tissé) qui sera la partie réhabitable, épaisseur 0,4 à 3mm.

L'ensemble ainsi préparé fait l'objet d'une cuisson à une température d'environ 150° pendant 10mn afin de polymériser la silicone

La feuille composite VERRE + TEFLON (12) est ensuite retirée afin de laisser le film de silicone (11) sur le textile poreux (10).

On peut réaliser simultanément deux plaques, en déposant sur chacune des faces de la feuille recouverte d'un film de silicone un textile poreux (10). Lorsque l'on sépare l'ensemble ainsi réalisé, on récupère deux plaques souples.

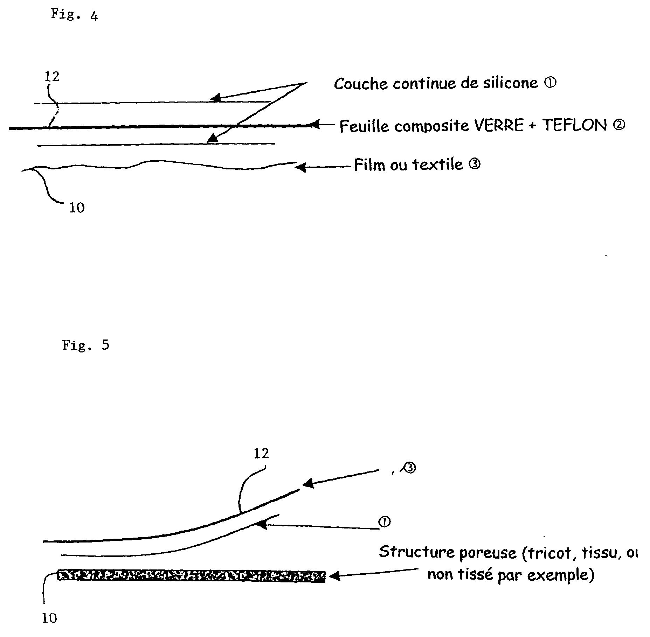

Les figures 4 et 5 se rapportent à un procédé de collage d'un film ou d'un textile microporeux sur un textile pour réaliser une plaque souple selon l'invention.

Le but de ce procédé est de coller un film très fin donc très souple, continu et lisse donc anti-adhérent ou un textile très fin sur un textile support (tissé, tricot, non tissé) poreux (10). (Exemple : non tissé trou 0,1 à 0,3mm - Tricot trou 0,5 à 2mm).

Le procédé consiste à réaliser une solution de silicone en dispersion dans du trichloroéthane 1,1,1 à raison de 60 grs de silicone pour 100 cm3 de trichloréthane. La silicone employée est de marque NUSIL type MED 42-11 par exemple et présente deux parties miscibles.

L'étape suivante consiste à tremper une feuille composite VERRE + TEFLON (12) de marque commerciale FLUOROCOMPOSITE ou BALTEX d'une épaisseur de 250 micromètres dans la solution réalisée à l'étape précédente afin d'obtenir après évaporation du trichloréthane 1,1,1 un film continu d'environ 300 micromètres de silicone.

On dépose ensuite la feuille composite enduite de silicone sur un film de polyéthylène téréphtalate (polyester = PES), polybutylène téréphtalate PBT, polytrétrafluoroéthylène PTFE (ou une autre matière thermoplastique ou thermodurcissable) ou sur un textile (tricot, tissu, non tissé). Le film doit être très fin (25 à 100 micromètre pour les PES (Société SYMPATEX), PBT (Société SYMPATEX), 50 à 200 micromètre pour le PTFE, le textile doit être très fin (50 à 100 micromètres et à pores très petits 5 à 10 micromètres) (Société DIATEX ou SEPHAR).

La feuille composite (2) est ensuite retirée afin de laisser la couche continue de silicone (4) sur le film ou textile (6). On dépose ensuite le film ou le textile (6) recouverte de sa couche de silicone (4) sur une structure macroporeuse (tricot, tissu ou non tissé), côté siliconé sur la structure macroporeuse.

L'ensemble fait l'objet d'une cuisson à 150° pendant 10mn.

Il va de soi que de nombreuses variantes peuvent être apportées, notamment par substitution de moyens techniques équivalents, sans sortir pour cela du cadre de l'invention.

## Revendications

1. Plaque chirurgicale souple composée d'un complexe multicouche comprenant une surface favorisant l'adhérence d'un tissu biologique et une surface non adhérente caractérisée en ce qu'elle est constituée par une couche extérieure constituée par un textile macroporeux tissé, tricoté ou non-tissé, ou grille extrudée à l'exclusion d'un matériau uniforme perforé, revêtu par une surface non adhérente.

2. Plaque chirurgicale selon la revendication 1 caractérisée en ce que la couche textile (2) est une couche tricotée présentant des trous compris entre 0,5 et 2 mm.

3. Plaque chirurgicale selon la revendication 1, caractérisée en ce que la couche textile (2) est une couche non-tissée présentant des trous compris entre 0,1 et 0,3 mm.

4. Plaque chirurgicale selon la revendication 1 caractérisée en ce que la couche textile (2) comprend des fibres résorbables et des fibres non résorbables.

5. Plaque chirurgicale selon la revendication 1 caractérisée en ce que la couche textile (2) est une couche tissée de fibres polyamides, polyester ou polyuréthane ou de polypropylène biocompatibles.

6. Plaque chirurgicale selon la revendication 1 caractérisée en ce que la couche textile (2) est une couche non-tissée de fibres polyamides, polyester ou polyuréthane ou de polypropylène biocompatibles.

7. Plaque chirurgicale selon la revendication 1 caractérisée en ce que la couche textile (2) est une couche tricotée de fibres polyamides, polyester ou polyuréthane ou de polypropylène biocompatibles.

8. Plaque chirurgicale souple composée d'un complexe multicouche selon l'une quelconque des revendications précédentes caractérisée en ce qu'il comprend une couche formée par un textile, une couche interne (1) constituée par un composé fluoré et une couche de liaison (3) continue constituée par un polymère thermoplastique ou thermodurcissable.

9. Plaque chirurgicale selon la revendication 8 caractérisée en ce que la couche de liaison (3) est constituée par un polymère de silicone.

10. Plaque chirurgicale selon l'une quelconque des revendications précédentes caractérisée en ce que la couche interne (1) est constituée de PTFE.

11. Plaque chirurgicale selon la revendication 1 caractérisée en ce que la couche de liaison polymérise à température ambiante.

12. Plaque chirurgicale selon la revendication 1 caractérisée en ce que la couche de liaison polymérise à une température comprise entre 0 et 230°C.

13. Plaque chirurgicale souple composée d'un complexe multicouche selon l'une quelconque des revendications 1 à 7 caractérisée en ce qu'il comprend une couche formée par un textile revêtu par une couche uniforme de silicone.

14. Procédé de fabrication d'une plaque chirurgicale souple composée d'un complexe multicouche comprenant une surface favorisant l'adhérence d'un tissu biologique et une surface non adhérente consistant à réaliser une solution de silicone en dispersion dans du trichloroéthane 1,1,1 à raison de 60 grs de silicone pour 100 cm3 de trichloréthane, à tremper une feuille composite VERRE + TEFLON dans la solution précitée afin d'obtenir un film continu d'environ 300 micromètres de silicone, à déposer la feuille composite enduite de silicone sur un textile, à réaliser la cuisson de l'ensemble à 150° pendant 10mn afin de polymériser la silicone, et à retirer la feuille composite VERRE + TEFLON afin de laisser le film de silicone sur le textile.

15. Procédé de fabrication d'une plaque chirurgicale souple composée d'un complexe multicouche selon la revendication 14 caractérisé en ce que l'on dépose sur chacune des surfaces de la feuille composite enduite de silicone un textile pour réaliser deux plaques simultanément, et en ce que l'on sépare après polymérisation les deux textiles et la feuille composite.

16. Procédé de fabrication d'une plaque chirurgicale souple composée d'un complexe multicouche comprenant une surface favorisant l'adhérence d'un tissu biologique et une surface non adhérente consistant à réaliser une solution de silicone en dispersion dans du trichloroéthane 1,1,1 à raison de 60 grs de silicone pour 100 cm3 de trichloréthane, à tremper une feuille composite VERRE + TEFLON dans la solution précitée pour réaliser un film continu d'environ 300 micromètres de silicone, à déposer la feuille composite enduite de silicone sur un film ou sur un textile (tricot, tissu, non tissé) d'une épaisseur comprise entre 25 à 200 micromètres puis à retirer la feuille composite (5) afin de laisser la couche continue de silicone (4) sur le film ou textile (6) et à déposer le film ou le textile (6) recouverte de sa couche de silicone (4) sur une structure poreuse et à procédé à une cuisson à environ 150° du composite ainsi réalise.
